# FASCICULE DE BREVET EUROPEEN

(11) **EP 1 664 012 B1**
(45) Date de publication et mention de la délivrance du brevet: **01.04.2009**
(21) Numéro de dépôt: 04787448.2
(22) Date de dépôt: 24.09.2004
(51) Int. Cl.: C07D 311/72, C07D 313/08, A61K 31/335, A61K 31/355, A61P 25/28

(54) **DERIVES DE TOCOPHEROL A LONGUE CHAINE HYDROXYLEE UTILES COMME NEUROTROPHIQUES**
TOCOPHEROLDERIVATE MIT EINER LANGEN HYDROXYLIERTEN KETTE, DIE SICH ALS NEUROTROPHE MITTEL EINSETZEN LASSEN
TOCOPHEROL DERIVATIVES WITH A LONG HYDROXYLATED CHAIN, WHICH CAN BE USED AS NEUROTROPHICS

(30) Priorité: 26.09.2003 FR 0311325
(43) Date de publication de la demande: 07.06.2006
(73) Titulaire: UNIVERSITE LOUIS PASTEUR, 67000 Strasbourg (FR); CENTRE NATIONAL DE LA RECHERCHE SCIENTIFIQUE (CNRS), 75794 Paris Cedex 16 (FR); Université du Luxembourg, 1511 Luxembourg-Limpertsberg (LU)
(72) Inventeur: LUU, Bang, F-67000 Strasbourg (FR); HEUSCHLING, Paul, L-8355 Garnich (LU); MULLER, Thierry, L-9026 Ettelbruck (LU); MORGA, Eleonora, L-1316 Luxembourg (LU)
(74) Mandataire: Kihn, Pierre Emile Joseph
(86) Numéro de dépôt international: PCT/FR2004/002424
(87) Numéro de publication internationale: WO 2005/030748

(56) Documents cités:
- WO-A-94/11343
- WO-A-03/068741
- US-A1- 2002 006 954

## Description

La présente invention a pour objet tout composé de formule (I) isolé ou synthétique, capables de moduler la spécification cellulaire des cellules souches neurales (modulation du ratio neurone/cellules gliales), de favoriser la différenciation puis la survie des neurones et cellules gliales en différenciation ainsi que la différenciation de cellules précurseurs d'oligodendrocytes en oligodendrocytes matures. Les composes selon l'invention sont par ailleurs capables de diminuer la composante inflammatoire lors de maladies affectant le système nerveux notamment en diminuant l'activation de la microglie et/ou des astrocytes et/ou en diminuant la gliose réactionnelle. L'invention concerne également les compositions comprenant ces composés ainsi que les méthodes de préparation de tels composés et leur utilisation dans le cadre de la préparation d'une composition pharmaceutique destinée à la prévention ou au traitement de maladies affectant le système nerveux.

Le système nerveux central (SNC) est constitué de deux populations cellulaires que l'on peut qualifier de cellules neurales : les neurones, et tous les autres types cellulaires, regroupés sous le nom de cellules gliales. Les astrocytes et les oligodendrocytes représentent les types majeurs des cellules gliales chez l'adulte. Ces cellules sont générées tout au long de la vie foetal et leur production se poursuit après la naissance et jusque chez l'adulte dans certaines régions du cerveau.
Neurones, astrocytes et oligodendrocytes dérivent d'un précurseurs commun, une cellule multipotentielle aux capacités de prolifération théoriquement illimitées, qui est localisée dans le tube neural. Ces précurseurs peuvent être considérés comme des cellules souches neurales car ils répondent aux critères qui définissent les cellules souches : auto-renouvellement, capacité de prolifération quasi infinie et capacité de générer les types cellulaires constituant le tissu dont elles sont issues.
Le neurone, cellule hyper spécialisée, se développe au sein d'un tissu de soutien et d'environmment, la glie. La glie centrale est composée des cellules gliales du système nerveux central et, la glie périphérique, des cellules gliales du système nerveux périphérique. Plus précisément, la glie centrale comprend les astrocytes (astroglie), les oligodendrocytes (oligodendroglie) et les microgliocytes (microglie). La glie périphérique comprend quant à elle les cellules de Schwann, équivalant des oligodendrocytes de la glie centrale.
Les astrocytes sont des constituants de la barrière hématoencéphalique (BHE). Ils interviennent dans la régulation du métabolisme cérébral et servent d'interface entre les capillaires et les neurones (rôle nutritif) par l'intermédiaire de prolongements (pseudopodes) qui s'enroulent autour des capillaires. Ils participent à la recapture des neurotransmetteurs et interviennent également dans la cicatrisation en produisant des filaments gliaux (constituants du cytosquelette).
Les microgliocytes sont des cellules gliales originaires de la lignée myéloide envahissant le système nerveux central pendant la période embryonnaire. La microglie est spécialisée dans l'élimination des macromolécules, la phagocytose des cellules en apoptose ou en nécrose, la reconnaissance et l'élimination d'éléments pathogènes, ainsi que dans la régulation de la réponse immunitaire.
Les oligodendrocytes assurent la myélinisation des axones dans le système nerveux central. Il existe une diversité de progéniteurs à l'origine des oligodendrocytes. Ces cellules sont générées dans des foyers ventriculaires très restreints tout au long du tube neural. Chez l'adulte les oligodendrocytes sont dispersés dans tout le parenchyme cérébral, avec une prédominance dans les faisceaux de substance blanche.

La gaine de myéline, synthétisée par l'oligodendrocyte, est un complexe protéique membranaire qui s'enroule autour des axones. Elle possède une double fonction: elle joue un rôle d'isolant électrique et surtout elle permet d'augmenter la vitesse de propagation de l'influx nerveux. Une atteinte de cette gaine conduira à un ralentissement, voire un arrêt du potentiel d'action, perturbant la transmission nerveuse de l'information et provoquant des troubles neurologiques. La perte ou le mauvais état de la myéline entraîne l'apparition de maladies dites démyélinisantes ou dysmyélinisantes. La plus répandue et la plus dévastatrice de ces maladies est la sclérose en plaques (SEP).

La sclérose en plaques est une maladie neurologique du jeune adulte qui associe une démyélinisation à des éléments inflammatoires, voire immunologiques. Les traitements actuels prennent relativement bien en compte la composante inflammatoire. Cependant ils s'avèrent inopérants sur la composante démyélinisation, cause du handicap permanent et cumulatif Contrairement à ce que l'on pensait autrefois, il s'avère que, même au début de la maladie, les oligodendrocytes gardent la potentialité de fabriquer de la nouvelle myéline (remyélinistation). Par contre, au stade chronique cette capacité de remyélinisation semble bien perdue. On sait ainsi que chez la plupart des malades, la SEP évolue d'abord sous la forme à "poussées et rémissions", ensuite sous la forme "progressive". Il semble bien que les oligodendrocytes et leurs précurseurs peuvent survivre aux phénomènes inflammatoires de la première phase, mais que par contre leur nombre et leur efficacité sont considérablement réduits pendant la phase chronique. Deux possibilités thérapeutiques étaient jusque là envisagées : la transplantation de précurseurs d'oligodendrocytes (greffe) ou une stimulation de la myélinisation par des substances chimiques à partir des oligodendrocytes survivants et des précurseurs d'oligodendrocytes endogènes (remyélinisation endogène).

En ce qui concerne la transplantation, l'utilisation des précurseurs des oligodendrocytes (cellules jeunes peu différenciées) considérés comme ayant de plus grandes possibilités de synthèse de myéline et de migration que les oligodendrocytes adultes, est envisagée dans l'art antérieur pour permettre la réparation d'un volume maximum de zones démyélinisées. La source idéale d'oligodendrocytes serait du tissu nerveux humain très jeune (embryonnaire) ce qui soulève des problèmes éthiques et pratiques. On ignore d'autre part quelles seront leurs propriétés migratoires. Or, le problème est que, chez la plupart des patients, les lésions sont multiples et qu'il est illusoire de les "greffer" chacune individuellement. On ne sait en outre toujours pas si ces oligodendrocytes sont capables de migrer par eux-mêmes ou si l'intervention de facteurs chimiques spécifiques est indispensable, de même que l'on ignore la longévité de ces cellules.

On a par ailleurs décrit, dans l'art antérieur, différentes substances peptidiques identifiées ci-dessus sous le nom de facteurs neurotrophiques (Recent progress in the studies of neurotrophic factors and their clinical implications, L. Shen, A. Figurov & B. Luu, Journal of Molecular Medicine, 1997, vol. 75, 637-644). Ce sont des facteurs de croissance spécifiques du cerveau. Ils protègent les cellules nerveuses contre diverses agressions, en particulier contre les substances libérées par les cellules microgliales activées et responsables de l'inflammation du cerveau ainsi que vis-à-vis de diverses maladies dues au mauvais fonctionnement du système nerveux. En règle générale, ces facteurs neurotrophiques augmentent la survie des cellules nerveuses, favorisent leur différenciation c'est-à-dire leur maturation, et les rendent fonctionnelles.

Dans le cadre de la présente invention, les inventeurs se sont penchés sur les actions possibles de nouveaux composés sur les cellules dites souches (Stem cells-Clinical application and Perspectives. M. Brehm, T. Zeus & B. E. Strauer, Herz, 2002, vol.27, 611-620). En effet, de récentes découvertes laissent entrevoir qu'un important chapitre est en train de s'ouvrir en biomédecine. Ces cellules souches pourraient être des outils indispensables pour une nouvelle branche de la médecine connue sous le nom de médecine régénératrice. (Stem cells for regenerative medicine : advances in the engineering of tissues and organs, J. Ringe, C. Kaps, G.R. Burmester & M. Sittinger, 2002, vol.89, 338-351) (Regenerating the damaged central nervous system, P.J. Horner & F.H. Gage, Nature, 2000, vol ; 407, 963-970). Cette médecine trouverait de puissantes applications dans le traitement des maladies liées à la vieillesse, des neuropathies dégénératives (Human stem cells as targets for the aging and diseases of aging processes, Medical Hypotheses, 2003, vol. 60, N° 3, 439-447) et des affections du système nerveux post traumatiques.

Les mécanismes par lesquels une cellule souche neurale donne naissance aux trois types cellulaires majeurs du SNC, sont encore mal connus. On sait cependant que ce développement procède par étapes successives au cours desquelles les potentialités développementales de la cellule souche neurale sont progressivement restreintes. Il y a ainsi production de précurseurs intermédiaires aux potentialités de plus en plus limitées qui aboutiront à des cellules hautement différenciées. Initialement, les cellules souches sont d'origine embryonnaire. Elles sont ainsi essentiellement présentes chez les foetus et les enfants en bas âge. Elles sont capables de se multiplier quasiment à l'infini. Sous l'action de ces facteurs neurotrophiques, elles se transforment en cellules matures et fonctionnelles de différents types (Neurons and astrocytes secrète factors that cause stem cells to differentiate into neurons and astrocytes, respectively, M.Y. Chang, H. Son, Y.S. Lee & S.H. Lee, Molecular and Cellular Neuroscience 2003, vol.23 N°3, 414-426). Très récemment, il a été démontré que les cellules souches sont aussi présentes dans des organes de stades plus développés, en particulier dans le cerveau (Adult Neurogenesis and Neural Stem Cells of the Central Nervous System in Mammals, Ph. Taupin & F.H. Gage, Journal of Neuroscience Research, 2002, vol. 69, 745-749) et la moelle épinière des adultes. Ainsi donc, sous l'action de différents facteurs de croissance, les cellules souches neurales, c'est-à-dire les cellules souches présentes dans le cerveau, peuvent se transformer en neurones, en astrocytes ou en oligodendrocytes, c'est-à-dire les principaux types de cellules nerveuses.

Cependant à cause de leur taille moléculaire et de leurs propriétés physicochimiques, ces facteurs de croissance naturels protéiques ne peuvent traverser diverses barrières biologiques, en particulier la barrière hématoencephalique. Ils ne peuvent donc pas pénétrer dans le cerveau en quantités suffisantes pour exercer leurs actions bénéfiques. En outre, ils présentent une très mauvaise biodisponibilité, limitant ainsi leur efficacité et leur utilisation.
La demande de brevet internationale WO03/06874 A1 décrit un composé comprenant un cycle indole portant un substituent alcoolique à longue chaine linéaire en position 3 par rapport à l'atome d'azote. Dans l'exemple test 1, le 3-(16-hydroxyhexadecyl) indole a été testé en relation avec l'effet sur la croissance de neurites et sur la survie des neurones
La demande de brevet internationale WO94/11343 décrit des alcools gras à longue chaîne comprenant un seul cycle non aromatique à six membres substitué par des groupes méthyles ayant une action neurotropique et promnesiante.
La demande de brevet US2002/0006954 A1 concerne et divulgue l'usage du gamma tocophérol et de tocophérols démethylés pour inhiber, bloquer ou désactiver l'azote réactif et d'autres espèces dans des tissus exposés à une inflammation ou à un autre stress, plus particulièrement dans du tissu du cerveaux exposé à un stress dû à l'azote.

L'invention propose à présent une alternative avantageuse à la transplantation qui consiste en l'utilisation de nouveaux composés. Ces derniers sont capables de franchir la barrière hématoencéphalique et de favoriser une remyélinisation « endogène » en modulant la specification cellulaire des cellules souches neurales (modulation du ratio neurone/cellules gliales) et/ou en favorisant la différentiation des précurseurs d'oligodendrocytes en oligodendrocytes. La présente invention décrit en effet la mise au point de petites molécules hydrophobes capables d'une part de pénétrer dans le cerveau en quantité suffisante pour promouvoir un effet biologique recherché et d'autre part, de mimer l'action de certains facteurs neurotrophiques. Ces mimes sont en mesure de transformer des cellules souches neurales en cellules nerveuses différenciées de même que des précurseurs d'oligodendrocytes en oligodendrocytes et ceci, in situ, dans le cerveau. Cette alternative permet ainsi d'éviter l'étape risquée de l'opération chirurgicale.
En effet, lorsque des cellules souches sont utilisées pour le traitement des neuropathies dégénératives, ces dernières sont introduites dans le cerveau à l'aide d'une opération chirurgicale (Neural stem cells in the developing central nervous system : implications for cell therapy through transplantation, C.N. Svendsen & M.A. Caldwell, Progress in Brain Research, 2000, vol. 127, 13-34) tout comme peuvent l'être les précurseurs des oligodendrocytes.

Les inventeurs ont ainsi mis au point et synthétisé des composés capables de moduler, *in vivo*, *ex vivo* ou *in vitro* la spécification cellulaire des cellules souches neurales, i.e., d'influencer le choix pour une cellule souche neurale de s'orienter vers la voie neuronale
ou gliale (modulation du ratio neurone/cellules gliales). Ces composés sont par ailleurs capables de favoriser la différenciation puis la survie des neurones et cellules gliales en différenciation. Plus particulièrement, les composés selon l'invention favorisent la survie neuronale et la croissance des neurites. Les composés selon l'invention sont également capables de favoriser la différenciation de cellules précurseurs d'oligodendrocytes en oligodendrocytes matures. Les composés selon l'invention sont aussi capables de diminuer la composante inflammatoire lors de maladies affectant le système nerveux. Ils peuvent ainsi, en particulier, diminuer l'activation de la microglie et/ou des astrocytes. Ces composés sont par ailleurs capables de diminuer la gliose réactionnelle, i.e., la cicatrisation gliale, plus particulièrement en modulant l'expression de certains composés du cytosquelette des astrocytes.

Un premier objet de l'invention concerne ainsi tout composé de formule (I) isolé ou synthétique provoquant, *in vivo, in vitro* ou *ex vivo,* la modulation de la spécification des cellules souches neurales et/ou la différentiation de cellules précurseurs d'oligodendrocytes en cellules oligodendrogliales et/ou réprimant l'activation des cellules microgliales et/ou l'activation des astrocytes et/ou la gliose réactionnelle.

De manière préférée, l'invention concerne tout composé de formule (I) isolé ou synthétique provoquant, *in vivo*, *in vitro* ou *ex vivo,* la modulation de la spécification de cellules souches neurales, et/ou la différentiation de cellules précurseurs d'oligodendrocytes en cellules oligodendrogliales, et/ou réprimant l'activation des cellules microgliales et/ou l'activation des astrocytes ainsi que, éventuellement, la gliose réactionnelle. Il concerne également l'utilisation d'un tel composé *in vivo* pour moduler, do préférence réprimer, l'activation des cellules microgliales, à l'instar des composés anti-inflammatoires non-stéroïdiens (NSAIDs), ces derniers n'ayant toutefois pas, au contraire des composés ou compositions selon l'invention, la capacité de franchir la barrière hématoencéphalique.

Les composés selon l'invention sont d'excellents agents pour le traitement des maladies neurodégéneratives ou démyélinisantes/dysmyélinisantes, telles que notamment la sclérose en plaques, la maladie d'Alzheimer, la maladie de Parkinson, la maladie de Creutzfeldt-Jakob, mais également les démences vasculaires, la sclérose latérale amyotrophiques, les amyotrophies spinales infantiles et les neuropathies liées aux accidents vasculaires cérébraux. L'invention concerne également leurs procédés de préparation et les compositions pharmaceutiques les contenant.

La présente invention a pour objet en particulier les alcools hydrocarbonés à chaîne longue, substitués par un noyau de type tocophérol, de formule (I) ainsi que leurs analogues. Ces composés sont nommés Tocopherol Fatty Alcohols (TFA).

Les composés selon l'invention comprennent de façon préférée une longue chaîne de ω-alcanol et un noyau de type tocophérol Avec une longueur de chaîne de taille appropriée et des substituants convenablement choisis, de tels composés sont capables de moduler la spécification cellulaire dans les cellules souches neurales, de favoriser la différenciation, la survie neuronale et la croissance neuritique ainsi que la différentiation et la survie des cellules oligodendrogliales. Comme indiqué précédemment, ces composés sont également capables de diminuer la composante inflammatoire qui survient lors de maladies affectant le système nerveux. Plus particulièrement, ces composés peuvent diminuer ou réprimer l'activation de la microglie ainsi que celle des astrocytes. Ces composés peuvent également diminuer la gliose réactionnelle, plus particulièrement en modulant l'expression de certains composés du cytosquelette des astrocytes. La répression de l'activation des cellules microgliales et la transformation des cellules souches neurales en cellules oligodendrocytaires matures sont des caractéristiques essentielles pour le traitement des maladies neurodégénératives ou de type démyélinisante/dysmyélinisante comme la sclérose en plaques.

Un objet particulier de l'invention concerne ainsi des composés représentés par la formule (I) générale suivante : 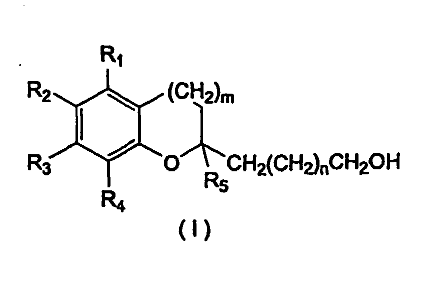 dans laquelle
- R₁, R₂, R₃ et R₄, identiques ou différents, représentent un atome d'hydrogène, un groupe hydroxyle, un groupe (C₁-C₆) alkyle, linéaire ou ramifié, un groupe (C₁-C₆) alkoxy linéaire ou ramifié, un groupe (C₁-C₆) carboxylate, linéaire ou ramifié,
- R₅ représente un atome d'hydrogène ou un groupe (C₁-C₆) alkyle linéaire ou ramifié,
- m est un nombre entier compris entre 0 et 2, de préférence entre 1 et 2, et
- n est un nombre entier compris entre 8 et 25, de préférence entre 8 et 20, encore plus préférentiellement entre 8 et 16 ou entre 8 et 14.

La formule (I) est constituée donc d'un noyau aromatique accolé à un cycle à 5, 6 ou 7 chaînons (m = 0, 1, 2), c'est-à-dire, un benzofurane, un benzopyrane ou l'un de ses analogues supérieurs. Il s'agit de préférence d'un benzopyrane. De préférence, m est égal à 1.

Les composés de formule (I) incluent les composés où l'atome de carbone portant le substituant R₅ est de configuration R, S ou un mélange (de préférence racémique).

Selon l'invention, le terme "alkyle" désigne un radical hydrocarboné linéaire ou ramifié ayant avantageusement de 1 à 6 atomes de carbone, tel que méthyle, éthyle, propyle, isopropyle, butyle, isobutyle, *tert*-butyle, pentyle, néopentyle, n-hexyle, etc. Lorsqu'au moins un R₁, R₂, R₃ et R₄ représente un radical alkyle, les groupes méthyle, éthyle, isopropyle ou tertiobutyle sont préférés.

Les groupes «alkoxy» correspondent aux groupes alkyle définis ci-dessus reliés au reste de la molécule par l'intermédiaire d'une liaison -O- (éther). On préfère tout particulièrement les groupes en C₁-C₆, notamment méthoxy, éthoxy, isopropoxy ou un tert-butoxy.

Les groupes «carboxylate» correspondent aux groupes -OCO-alkyle, le terme alkyle étant tel que définis ci-dessus, tels qu'un acétate, un propionate, un butylate, un pentanoate ou un hexanoate.

Selon un aspect particulier de l'invention, les composés de formule (I) correspondent à ceux pour lesquels au moins un, de préférence un seul, des substituants présents sur le noyau aromatique, R₁, R₂, R₃ et/ou R₄ représente un groupe hydroxyle, un groupe alkoxy ou carboxylate.

Selon un autre aspect particulier de l'invention, R₅ représente un atome d'hydrogène ou un groupe alkyle, de préférence méthyle, avec une configuration R, S ou un mélange (de préférence racémique).

La chaîne latérale du composé de formule (I) correspond donc à un ω-alcanol dans lequel n est compris entre 8 et 25, de préférence entre 8 et 20, encore plus préférentiellement entre 8 et 16 ou entre 8 et 14. Les composés de formule (I) dans lesquels n est égal à 10, 11, 12, 13, 14, 15 ou 16 sont des composés particulièrement efficaces dans le cadre de la présente invention. Les composés comprenant une chaîne latérale d'au moins 12 atomes de carbone et au plus 18 atomes de carbone, i.e. TFA-12, TFA-14, TFA-15, TFA-16 et TFA-18, sont particulièrement préférés. D'autres composés, pouvant être synthétisés selon un mode opératoire tel que décrit ci-dessous, dans lesquels R₁, R₂, R₃ et R₄ sont des groupements méthoxy ou des groupements acétate, leur chaîne latérale ayant les mêmes nombres d'atomes de carbones que précédemment décrit, sont également efficaces.

Un objet de l'invention concerne ainsi une composition pharmaceutique comprenant à titre de substance active au moins un composé de formule (I) isolé ou synthétique capable de moduler *in vivo*, *in vitro* ou *ex vivo* la spécification cellulaire des cellules souches neurales, de favoriser *in vivo*, *in vitro* ou *ex vivo* la différenciation de cellules souches neurales et/ou de cellules précurseurs d'oligodendrocytes en cellules oligodendrogliales et la survie desdites cellules oligodendrogliales ou au contraire de favoriser la différentiation de cellules souches neurales en neurones et la survie desdits neurones, ou encore de moduler, de préférence réprimer, l'activation des cellules microgliales et/ou l'activation des astrocytes, et/ou la gliose réactionnelle, en association avec un véhicule acceptable sur le plan pharmaceutique. Une composition pharmaceutique préférée, au sens de l'invention, comprend à titre de substance active au moins un composé de formule (I) isolé ou synthétique capable de moduler la spécification de cellules souches neurales et/ou de provoquer la différentiation de cellules précurseurs d'oligodendrocytes en cellules oligodendrogliales, et/ou la modulation, de préférence la répression ou la diminution, de l'activation de cellules microgliales et/ou d'astrocytes, et/ou de moduler, de préférence réprimer, la gliose réactionnelle, en association avec un véhicule acceptable sur le plan pharmaceutique.

Un autre objet de la présente invention concerne également des compositions pharmaceutiques comprenant à titre de substance active au moins un composé répondant à la formule générale (I) selon l'invention, telle que définie précédemment en association avec un véhicule ou un excipient acceptable sur le plan pharmaceutique.

Quelle que soit la voie d'administration choisie, des compositions préférées selon l'invention se présentent sous une forme favorable à la protection et à l'assimilation optimale du principe actif.

Les composés ou compositions selon l'invention peuvent être administrés de différentes manières et sous différentes formes. Ainsi, ils peuvent être administrés de manière systémique, par voie orale, par inhalation ou par injection, comme par exemple par voie intraveineuse, intra-musculaire, sous-cutanée, trans-dermique, intra-artérielle, etc., les voies intraveineuse, infra-musculaire sous-cutanée, orale et par inhalation étant préférées. Pour les injections, les composés sont généralement conditionnés sous forme de suspensions liquides, qui peuvent être injectées au moyen de seringues ou de perfusions, par exemple A cet égard, les composés sont généralement dissous dans des solutions salines, physiologiques, isotoniques, tamponnées, etc., compatibles avec un usage pharmaceutique et connues de l'homme du métier. Ainsi, les compositions peuvent contenir un ou plusieurs agents ou véhicules choisis parmi les dispersants, solubilisants, stabilisants, conservateurs, etc. Des agents ou véhicules utilisables dans des formulations liquides et/ou injectables sont notamment la méthylcellulose, l'hydroxyméthylcellulose, la carboxyméthylcellulose, le polysorbate 80, le mannitol, la gélatine, le lactose, des huiles végétales, l'acacia, etc.
Les composés peuvent également être administrés sous forme de gels, huiles, comprimés, suppositoires, poudres, gélules, capsules, aérosols, etc., éventuellement au moyen de formes galéniques ou de dispositifs assurant une libération prolongée et/ou retardée. Pour ce type de annulation, on utilise avantageusement un agent tel que la cellulose, des carbonates ou des amidons.
Les composés selon l'invention peuvent en particulier moduler l'activation des cellules microgliales et astrocytaire aux concentrations de 10⁻⁵ à 10⁻¹², de préférence 10⁻⁶ à 10⁻⁸M, encore plus préférentiellement 10⁻⁵ à 10⁻⁸M. Ces composés transforment, dans les mêmes conditions préférées de concentrations, les cellules précurseurs d'oligodendrocytes en oligodendrocytes matures. Les composés selon l'invention peuvent également favoriser la differenciation et la survie des neurones dans ces conditions.

Les inventeurs ayant démontré une activité des composés selon l'invention aux concentrations indiquées ci-dessus, il est entendu que le débit et/ou la dose injectée peuvent être adaptés par l'homme du métier en fonction du patient, de la pathologie concernée, du mode d'administration, etc. En outre, des injections répétées peuvent être réalisées, le cas échéant. D'autre part, pour des traitements chroniques, des systèmes retard ou prolongés peuvent être avantageux.

L'invention concerne par ailleurs l'utilisation d'un composé de formule I dans le cadre de la préparation d'une composition pharmaceutique destinée à la prévention ou au traitement des maladies du système nerveux, altérant les oligodendrocytes ou les autres cellules du système nerveux, et/ou de l'inflammation du système nerveux. De telles maladies incluent notamment les neuropathies dégénératives, et en particulier les maladies démyélinisantes ou dysmyelinisantes ainsi que la maladie d'Alzheimer,

Au sens de l'invention, le terme traitement désigne aussi bien un traitement préventif que curatif, qui peut être utilisé seul ou en combinaison avec d'autres agents ou traitements. En outre, il peut s'agir d'un traitement de troubles chroniques ou aiguës.

Ainsi donc les composés TFA tels que décrits ci-dessus, peuvent être utilisés comme agents pharmaceutiques dans le traitement des maladies neurodégénératives et des maladies démyélinisantes, en particulier de la sclérose en plaques.

Un autre objet de l'invention concerne l'utilisation d'un composé ou d'une composition selon l'invention qui induit *in vivo* la modulation de la spécification cellulaire des cellules souches neurales, la différenciation et la survie des neurones et des oligodendrocytes, la modulation de l'activation microgliale et astrocytaire ainsi que de la gliose réactionnelle.

Un autre objet de l'invention concerne l'utilisation d'un composé ou d'une composition selon l'invention pour le traitement, *ex vivo,* de cellules souches ou de cellules précurseurs d'oligodendrocytes, telles que décrites précédemment En particulier ce traitement consiste à induire la spécification et/ou la différenciation desdites cellules.

L'invention concerne aussi une méthode de traitement préventif ou curatif des maladies du système nerveux altérant les oligodendrocytes ou leur activité et/ou de l'inflammation du système nerveux, comprenant l'administration à un sujet atteint d'une telle pathologie ou présentant un risque d'en développer, d'une quantité efficace d'un composé ou d'une composition selon l'invention, par exemple d'un composé répondant à la formule (I), ou encore de cellules souches ou de cellules précurseurs d'oligodendrocytes traitées *ex vivo,* comme indiqué ci-dessus.

Des maladies susceptibles d'être traitées à l'aide d'un composé selon l'invention sont en particulier la sclérose en plaques, la maladie d'Alzheimer, la maladie de Parkinson, la maladie de Creutzfeldt-Jakob. D'autres maladies susceptibles d'être traitées sont les démences vasculaires, la sclérose latérale amyotrophique, les amyotrophies spinales infantiles. De même les lésions dérivées d'accidents vasculaires cérébraux et toutes autres atteintes lésionnelles du système nerveux sont susceptibles d'être traitées par les composés et compositions de l'invention.

Les composés de l'invention peuvent être préparés à partir de produits du commerce, en mettant en oeuvre une combinaison de réactions chimiques connues de l'homme du métier, éventuellement à la lueur du procédé de préparation des composés de formule générale (I) tel que décrit ci-dessous.

Les composés de formule générale (I) peuvent être notamment obtenus par un procédé de préparation comprenant les étapes suivantes :
une formation d'un amide de Weinreb, une addition-élimination, une addition d'un magnésien et un couplage catalysé par ZnCl₂ et HCl.

Un composé de formule (I) selon l'invention peut, par exemple, être préparé selon le schéma réactionnel particulier suivant : 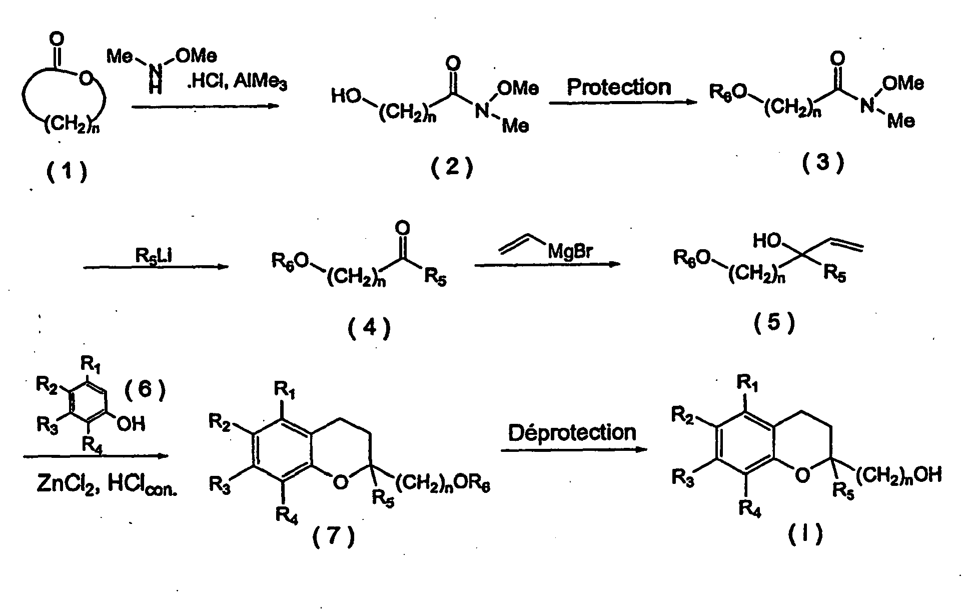 dans lequel :
- R₁, R₂, R₃, R₄, R₅ et n ont la même signification que décrite précédemment.
- R₅ représente un groupement benzyle ou un groupement *tert-*butyldiméthylsilyle.

Plus précisément, le composé (I) est préparé en faisant réagir le composé (1) avec la diméthylhydroxylamine en présence de triméthylaluminium pour obtenir le composé (2) dont la fonction alcool est ensuite protégée pour donner le composé (3). Une addition nucléophile d'un organolithien sur le composé (3) fournit le composé (4) qui, en présence de vinylmagnésien, donne le composé (5). La réaction entre le composé (5) et le composé (6), en présence de chlorure de zinc et d'acide chlorhydrique concentré, fournit le composé (7) qui, par déprotection de la fonction alcool, donne le composé (I).

Plus précisément, le composé (I) où R₁, R₃, R₄ et R₅ sont des groupements méthyles, R₂ est un groupement hydroxyle, m =1 et n =13 peut être préparé de la façon suivante : De l'oxacycloheptadécan-2-one réagit avec de la diméthylhydroxylamine en présence de triméthylaluminium à 0°C et à pression atmosphérique pour donner du 16-hydroxy-hexadécan-méthoxy-méthyl-amide. La fonction alcool de ce composé est ensuite protégée en présence d'hydrure de sodium et de bromure de benzyle à 78°C et à pression atmosphérique. Le 16-benzyloxy-hexadécan-méthoyx-méthyl-amide ainsi obtenu, est ensuite transformé par action du méthyllithium en 17-benzyloxy-heptadécan-2-one à -78°C et à pression atmosphérique. Ce dernier réagit avec du vinylmagnésien pour donner le 18-benzyloxy-3-méthyl-octadéc-1-èn-3-ol qui en présence de triméthylhydroquinone, de chlorure de zinc et d'acide chlorhydrique concentré, fournit le 2-(15-benzyloxy-pentadécyl)-2,5,7,8-tétraméthyl-chroman-6-ol. Ce composé est finalement soumis à une hydrogénation catalytique en présence de palladium sur charbon pour donner le 2-(15-hydroxy-pentadécyl)-2,5,7,8-tétraméthyl-chroman-6-ol.

L'invention concerne par ailleurs des outils et kits destinés à la mise en oeuvre de l'une ou l'autre des méthodes telles que décrites ci-dessus.

D'autres aspects et avantages de la présente invention apparaîtront à la lecture des exemples qui suivent, fournis à titre illustratif et non limitatif.

### EXEMPLES

### A. Préparation d'un composé de formule (1)

### 1. Préparation de 16-Hydroxy-hexadécan-méthoxy-méthyl-amide

1,78 g de diméthylhydroxylamine (17,687 mmol ; 3 éq. ; PM = 97,55) sont dissous dans 10 mL de CH₂Cl₂ distillé sous argon puis refroidis à - 78 °C. 8,8 mL de AlMe₃ à 2 M dans l'hexane (17,687 mmol ; 3 éq. ; PM = 72,09) sont ajoutés goutte à goutte à - 78 °C puis la réaction est laissée sous agitation à température ambiante pendant une demi-heure. La solution est ensuite refroidie à 0 °C et 1,55 g d'oxacycloheptadécan-2-one (5,895 mmol ; 1 éq. ; PM = 254,42) dissous dans 5 mL de CH₂Cl₂ distillé sont ajoutés goutte à goutte. La réaction est laissée sous agitation à température ambiante. Une analyse par chromatographie en couche mince montre que la réaction est terminée après une heure et demie. La solution est versée goutte à goutte dans un mélange de 60 mL de Et₂O/MeOH 2 :1 refroidi à -78 °C puis filtrée sur célite. 100 mL d'une solution saturée aqueuse de NaHCO₃ sont ajoutés et la phase aqueuse est extraite à l'éther. Les phases organiques réunies sont séchées par du sulfate de magnésium puis évaporées sous pression réduite pour donner 1,66 g de 16-hydroxy-hexadécan-méthoxy-méthyl-amide, ce qui correspond à 5,262 mmol, et à un rendement de 89 %. L'amide ainsi obtenu sera utilisé sans purification supplémentaire.
R_{f}= 0,1 ; éluant : 30 % d'acétate d'éthyle dans l'hexane
**RMN ¹H** (300 MHz, CDCl₃) δ : 1,24 (s large, 22H, H-4 à H-14) ; 1,52-1,63 (m, 4H, H-3 et H-15) ; 2,39 (t, 2H, J = 7,7 Hz, H-2) ; 3,16 (s, 3H, H-1') ; 3,61-3,66 (m, 5H, H-16 et H-2')
**RMN ¹³C** (75 MHz, CDCl₃) δ : 24,6 (CH₂, C-14) ; 25,7 (CH₂, C-3), 29,4-29,6 (10 x CH₂, C-4 à C-13) ; 31,9 (CH₂, C-2) ; 32,0 (CH₃, C-1') ; 32,8 (CH₂, C-15) ; 61,16 (CH₃, C-2'); 63,0 (CH₂, C-16)

### 2. Préparation de 16-Benzylozy-hexadécan-méthoxy-méthyl-amide

3,27 g de 16-hydroxy-hexadecan-méthoxy-méthyl-amide (10,364 mmol ; 1 éq. ; PM = 315, 49) sont dissous dans 40 mL de THF distillé sous agitation. 0,83 g de NaH à 60 % dans l'huile (20,729 mmol ; 2 éq. ; PM = 24) lavé à l'hexane est ajouté et la solution est chauffée sous reflux (75 °C) pendant une demi-heure. 1,48 mL de BnBr (12,437 mmol ; 1,2 éq. ; PM =171,04) sont ajoutés et la solution est chauffée sous reflux. Une analyse par chromatographie en couche mince montre que la réaction est terminée après 24 heures. 100 mL d'une solution aqueuse saturée de NH₄Cl sont ajoutés et la phase aqueuse est extraite à l'éther. Les phases organiques réunies sont séchées par du sulfate de magnésium et évaporées sous pression réduite pour donner un brut jaune. Le brut est chromatographié sur colonne de silice (5 x 15 cm, éluant 30 % d'acétate d'éthyle dans l'hexane). Au total, 2,92 g de 16-benzyloxy-hexadécan-méthoxy-méthyl-amide sont récupérés, ce qui correspond à 7,198 mmol et à un rendement de 69,4 %.
R_{f}= 0,5 ; éluant : 30 % d'acétate d'éthyle dans l'hexane
**RMN ¹H** (300 MHz, CDCl₃) δ : 1,25 (s large, 22H, H-4 à H-14) ; 1,56-1,65 (m, 4H, H-3 et H-15) ; 2,40 (t, 2H, J = 7,6 Hz, H-2) ; 3,17 (s, 3H, H-1") ; 3,46 (t, 2H, J = 6,6 Hz, H-16) ; 3,67 (s, 3H, H-2") ; 4,50 (s, 2H, H-17) ; 7,24-7,34 (m, 5H, H-2' à H-6')
**RMN ¹³C** (75 MHz, CDCl₃) δ : 24,6 (CH₂, C-15) ; 26,2 (CH₂, C-3) ; 29,3-29,7 (10 x CH₂, C-4 à C-14) ; 31,9 (CH₂, C-2); 32,1 (CH₃, C-1") ; 61,2 (CH₃,C-2") ; 70,5 (CH₂, C-16) ; 72,8 (Ph-CH₂, C-17) ; 127,4 (Ph, C-4') ; 127,6 (Ph, C-2' et C-6') ; 128,3 (Ph, C-3' et C-5') ; 138,7 (Ph, G1')

### 3. Préparation de 17-Benzyloxy-heptadécan-2-one

0,5 g de 16-benzyloxy-hexadécan-méthoxy-méthyl-amide (1,232 mmol ; 1 éq. ; PM = 405,62) est dissous dans 8 mL de THF distillé sous argon puis refroidi à - 78°C. 2,46 mL de MeLi à 1,5 M dans l'éther (3,698 mmol ; 3 éq. ; PM = 21,95) sont ajoutés goutte à goutte à -78 °C. Une analyse par chromatographie en couche mince montre que la réaction est instantanée. 100 mL d'une solution aqueuse saturée de NH₄Cl sont ajoutés et la phase aqueuse est extraite à l'éther. Les phases organiques réunies sont séchées par du sulfate de magnésium et évaporées sous pression réduite pour donner un brut jaune. Le brut est chromatographié sur colonne de silice (5 x 15 cm, éluant 15 % d'acétate d'éthyle dans l'hexane). Au total, 0,34 g de 17-benzyloxy-heptadécan-2-one est récupéré, ce qui correspond à 0,941 mmol et à un rendement de 76,3 %.
R_{f}= 0,6 ; éluant : 30 % d'acétate d'éthyle dans l'hexane
**RMN ¹H** (300 MHz, CDCl₃) δ : 1,26 (s large, 22H, H-5 à H-15) ; 1,55-1,65 (m, 4H, H-4 et H-16) ; 2,14 (s, 3H, H-1) ; 2,42 (t, 2H, J = 7,5 Hz; H-3) ; 3,47 (t, 2H, J = 6,6 Hz, H-17) ; 4,51 (s, 2H, H-18) ; 7,25-7,36 (m, 5H, H-2' à H-6')
**RMN ¹³C** (75 MHz, CDCl₃) δ : 23,9 (CH₂, C-4) ; 26,2-29,8 (13 x CH₂, C-1, C-5 à C-16) ; 43,8 (CH₂, C-3) ; 70,5 (CH₂, C-17) ; 72,8 (Ph-CH₂, C-18) ; 127,4 (Ph, C-4') ; 127,6 (Ph, C-2' et C-6') ; 128,3 (Ph,C-3' et C-5') ; 138,7 (Ph, C-1') ; 209,4 (C=O)

### 4. Préparation de 18-Benzyloxy-3-méthyl-octadéc-1-èn-3-ol

0,33 g de 17-benzyloxy-heptadécan-2-one (0,915 mmol ; 1 éq. ; PM = 360,6) est dissous dans 10 mL de THF distillé sous agitation puis refroidi à 0 °C. 2,75 mL de bromure de vinylmagnésien à 1 M dans le THF (2,745 mmol ; 3 éq. ; PM = g/mol) sont ajoutés goutte à goutte à 0 °C. La solution est laissée sous agitation à température ambiante. Une analyse par chromatographie en couche mince montre que la réaction est terminée après 3 heures. 100 mL d'une solution aqueuse saturée de NH₄Cl sont ajoutés et la phase aqueuse est extraite à l'éther. Les phases organiques réunies sont séchées par du sulfate de magnésium et évaporées sous pression réduite pour donner un brut jaune. Le brut est chromatographié sur colonne de silice (4 x 15 cm, éluant 20 % d'acétate d'éthyle dans l'hexane). Au total, 0,31 g de 18-benzyloxy-3-méthyl-octadéc-1-èn-3-ol est récupéré, ce qui correspond à 0,812 mmol et à un rendement de 88,7%.
R_{f} = 0,7 ; éluant : 30 % d'acétate d'éthyle dans l'hexane
**RMN ¹H** (300 MHz, CDCl₃) δ : 1,26 (s, 3H, H-3a) ; 1,27 (s large, 24H, H-5 à H-16) ; 1,54-1,64 (m, 4H, H-4 et H-17) ; 3,47 (t, 2H, J = 6,6 Hz, H-18) ; 4,51 (s, 2H, H-19) ; 5,04 (dd, 1H, J_{1a-1b}= 1,2 Hz, J₁ₐ₋₂ = 10,6 Hz, H₁ₐ) ; 5,20 (dd, 1H, J_{1b-1a}= 1,2 Hz, J_{1b-2} = 17,3 Hz, H_{1b}) ; 5,91 (dd, 1H, J₂₋₁ₐ = 10,6 Hz, J_{2-1b}= 17,3 Hz, H_{1b}) ; 7,26-7,35 (m, 5H, H-2' à H-6')
**RMN ¹³C** (75 MHz, CDCl₃) δ : 23,9 (CH₂, C-5) ; 26,2 (CH₂, C-6) ; 27,7 (CH₃, C-3a) ; 29,5-30,0 (11 x CH₂, C-7 à C-17) ; 42,4 (CH₂, C-4) ; 70,5 (CH₂, C-18) ; 72,8 (Ph-CH₂, C-19) ; 73,3 (C quaternaire, C-3) ; 111,4 (CH₂, C-1) ; 127,4 (Ph, C-4') ; 127,6 (Ph, C-2' et C-6') ; 128,3 (Ph, C-3' et C-5') ; 138,7 (Ph, C-1') ; 145,3 (CH, C-2)

### 5. Préparation de 2-(15-Benzyloxy-pentadécyl)-2,5,7,8-tétraméthyl-chroman-6-ol

0,11 g de triméthylhydroquinone (0,728 mmol ; 1 éq. ; PM = 152,19) est dissous dans 3 mL d'acétate d'éthyle. 0,08 g de ZnCl₂ (0,728 mmol ; 0,8 éq. ; PM = 136, 29) puis 0,01mL de HCl concentré (0,145 mmol ; 0,2 éq. ; PM = 36,46) sont ajoutés. Après 5 minutes à température ambiante, 0,28 g de 18-benzyloxy-3-méthyl-octadéc-1-èn-3-ol (0,728 mmol ; 1 éq. ; PM = 388,33) dissous dans 4 mL d'acétate d'éthyle, est ajouté goutte à goutte. Une analyse par chromatographie en couche mince, montre que la réaction est terminée après 48 h. 100 mL d'une solution aqueuse saturée de NaHCO₃ sont ajoutés et la phase aqueuse est extraite à l'éther. Les phases organiques réunies sont séchées par du sulfate de magnésium et évaporées sous pression réduite pour donner un brut rouge. Le brut est chromatographie sur colonne de silice (4 x 15 cm, éluant 15 % d'acétate d'éthyle dans l'hexane). Au total, 0,29 g de 2-(15-benzyloxy-pentadécyl)-2,5,7,8-tétraméthyl-chroman-6-ol est récupéré, ce qui correspond à 0,554 mmol et à un rendement de 76 %.
R_{f}= 0,74 ; éluant : 30 % d'acétate d'éthyle dans l'hexane
**RMN ¹H** (300 MHz, CDCl₃) δ : 1,22 (s, 3H, H-2a) ; 1,25 (s large, 24H, H-2' à H-13') ; 1,51-1,66 (m, 4H, H-1' et H-14'); 1,70-1,86 (m, 2H, H-3) ; 2,11 (s, 6H, H-5a, H-7a) ; 2,16 (s, 3H, H-8a) ; 2,6 (t, 2H, J = 6,8 Hz, H-4) ; 3,46 (t, 2H, J = 6,6 Hz, H-15') ; 4,18 (s, 1H, phénoxy) ; 4,50 (s, 2H, H-16') ; 7,27-7,35 (m, 5H, H-2" à H-6")
**RMN ¹³C** (75 MHz, CDCl₃) δ : 11,3 (CH₃, C-5a) ; 11,8 (CH₂, C-7a) ; 12,2 (CH₃, C-8a) ; 20,7 (CH₂, C-4) ; 23,6 (CH₂, C-2') ; 23,8 (CH₃, C-2a) ; 26,2 (CH₃, C-3') ; 29,5-30,0 (11 x CH₂, C-4' à C-14') ; 31,5 (CH₂, C-3) ; 39,5 (CH₂, C-1') ; 70,5 (CH₂, C-15') ; 72,8 (Ph-CH₂, C-16') ; 74,5 (C quaternaire, C-2) ; 117,3 (Ph, C-5) ; 118,4 (Ph, C-6) ; 121,0 (Ph, C-8) ; 122,6 (Ph, C-7) ; 127,4 (Ph, C-4") ;127,6 (Ph, G2" et C-6") ; 128,3 (Ph, C-3" et C-5") ; 138,7 (Ph, C-1") ; 144,5 (Ph, C-4a) ; 145,6 (Ph, C-8b)

### 6. Préparation de 2-(15-Hydroxy-pentadécyl)-2,5,7,8-tétraméthyl-chroman-6-ol

0,16 g de 2-(15-benzyloxy-pentadécyl)-2,5,7,8-tétraméthyl-chroman-6-ol (0,312 mmol ; 1 éq. ; PM = 522,52) est dissous dans 8 mL d'éthanol. 0,3 g de Pd/C (5 %) (20 % en masse) est ajouté sous argon puis l'argon est remplacé par du dihydrogène. En tout, 3 cycles vide-H₂ sont effectués. Une analyse par chromatographie sur couche mince montre que l'hydrogénation est totale après 4 heures. La solution est filtrée sur célite et évaporée sous pression réduite pour donner un brut blanc. Le brut est chromatographie sur colonne de silice (3 x 15 cm, éluant 40 % d'acétate d'éthyle dans l'hexane). Au total, 0,13 g de 2-(15-hydroxy-pentadécyl)-2,5,7,8-tétraméthyl-chroman-6-ol est récupéré, ce qui correspond à 0,3 mmol et à un rendement de 96,4 %.
R_{f}= 0,56 ; éluant : 30 % d'acétate d'éthyle dans l'hexane
**RMN ¹H** (300 MHz, CDCl₃) δ : 1,22 (s, 3H, H-2a) ; 1,25 (s large, 24H, H-2' à H-13') ; 1,51-1,61 (m, 4H, H-1' et H-14'); 1,70-1,86 (m, 2H, H-3) ; 2,11 (s, 6H, H-5a, H-7a); 2,16 (s, 3H, H-8a) ; 2,6 (t, 2H, J = 6,8 Hz, H-4) ; 3,64 (t, 2H, J = 6,6 Hz, H-15') ; 4,24 (s, 1H, phénoxy)
**RMN ¹³C** (75 MHz, CDCl₃) δ : 11,3 (CH₃, C-5a) ; 11,8 (CH₂, C-7a) ; 12,2 (CH₃, C-8a) ; 20,7 (CH₂, C-4) ; 23,6 (CH₂, C-2') ; 23,8 (CH₃, C-2a) ; 26,2 (CH₃, C-3') ; 29,5-30,0 (11 x CH₂, C-4' à C-14') ; 31,5 (CH₂, C-3) ; 39,5 (CH₂, C-1') ; 70,5 (CH₂, C-15'); 72,8 (Ph-CH₂, C-16') ; 74,5 (C quaternaire, C-2) ; 117,3 (Ph, C-5) ; 118,4 (Ph, C-6) ; 121,0 (Ph, C-8) ; 122,6 (Ph, C-7) ; 144,5 (Ph, C-4a) ;145,6 (Ph, C-8b)

Les composés dans lesquels R₂ est égal à un groupement méthoxy ou un groupement acétate (n étant égal à 13 et m étant égal à 1), ont été synthétisés de la même façon, R₁, R₃, R₄ et R₅ étant des groupements tels que définis précédemment dans le texte, en particulier méthyle. D'autres composés dans lesquels R₁, R₃ ou R₄ représente un groupement hydroxyle, méthoxy ou acétate, les autres substituants étant tels que définis précédemment dans le texte, en particulier méthyle, ont également été synthétisés.

### B. Inhibition de l'activation, de la microglie par les TFA (Tocopherol Fatty Alcohols dans lesquels R₁, R₃, R₄ et R₅ sont des groupes méthyle et R₂ correspond à un groupe hydroxyle)

Les expériences réalisées concernent la capacité de ces molécules à inhiber dans la microglie activée, la libération de nitrites et du Tumour Necrosis Factor-alpha (TNF-α). Une autre expérience réalisée visait à étudier l'expression du MHC IL

### 1. Mesures de la libération de nitrites

L'activité de la NO-synthase de type II (NOS II) représente un paramètre de l'activation microgliale souvent analysée dans la littérature. Cette enzyme est responsable de la synthèse du radical monoxyde d'azote en condition d'activation inflammatoire. Une activation de 24 à 48 heures entraîne une forte augmentation de l'expression de l'enzyme. Le produit de cette enzyme, le NO, se dégrade rapidement en culture pour former des nitrites. Un dosage, par colorimétrie (méthode de Griess), montre que les niveaux de NO₂⁻ suivent la même tendance.
Dans les expériences réalisées, les inventeurs ont mesuré, après 24 heures et 48 heures d'activation, les concentrations en NO2⁻, dans des cultures de cellules microgliales traitées avec 0.01 µg/ml de LPS, en présence des produits TFA-10, TFA-12, TFA-14, TFA-16 et de la vitamine E à des concentrations de 10⁻⁵, 10⁻⁶ et 10⁻⁷ M.
Les résultats obtenus provenant, des trois expériences indépendantes, montrent que les alcools gras TFA-10, TFA-12, TFA-14, TFA-16, à des concentrations de 10⁻⁵ M, entraînent une diminution de la production de nitrites par la microglie, diminution allant jusqu'à 50% par rapport aux valeurs contrôles. L'activité de ces produits est concentration dépendante puisqu'elle décroît avec la concentration. Les produits TFA-12, TFA-14, TFA-16 semblent présenter une activité plus forte que le TFA-10 à 10⁻⁵ M.
Ces résultats montrent que les produits selon l'invention sont capables de réduire la production de nitrites des cellules microgliales activées par le LPS. Ces résultats montrent qu'il existe un rapport entre la longueur de chaîne de ces produits et leurs activités, puisque le TFA-10 (qui correspond à la plus petite chaîne) possède la plus faible activité de ces alcools gras tocophéroliques.

### 2. Mesure de la libération de TNF-α

L'expression du TNF-α représente, un paramètre de l'activation microgliale souvent analysé dans la littérature. La microglie au repos n'exprime pas cette cytokine: Une activation de 24 heures par le LPS entraîne une forte augmentation de l'expression, détectable du TNF-α par test ELISA.
Dans les expériences réalisées; les inventeurs ont mesuré après 24 heures d'activation, les concentrations en TNF-α dans des cultures de cellules microgliales traitées avec 0.01 µg/ml de LPS en présence de TFA-10, TFA-12, TFA-14, TFA-16 et de vitamine E à des concentrations de 10⁻⁵, 10⁻⁶ et 10⁻⁷ M.
Les résultats obtenus lors d'expériences indépendantes les unes des autres concernant le TNF-α montrent que les alcools gras tocophéroliques TFA-12, TFA-14 et TFA-16 à 10⁻⁵ M entraînent une diminution dans les cellules de la production de TNF-α de 30 à 40% par rapport aux valeurs contrôles. L'activité de ces produits est concentration dépendante puisqu'elle décroît avec la concentration. Le produit TFA-10 ne présente pas d'activité, tout comme la vitamine E.
Ces résultats préliminaires sur la production de TNF-α confirment les résultats obtenus avec les expériences concernant la production de nitrites.
Par conséquent, ces résultats démontrent qu'il existe un rapport, au niveau des alcools gras tocophéroliques, entre la longueur de chaîne de ces produits et leurs activités.

## Revendications

1. Composés, **caractérisés en ce qu'**ils présentent la formule générale (I) suivante : 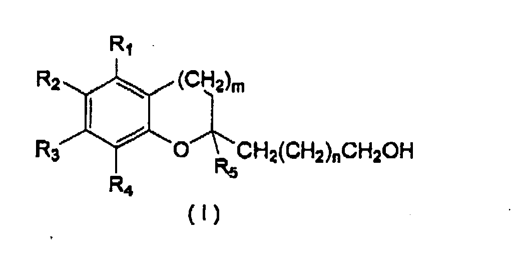 dans laquelle
- R₁, R₂, R₃ et R₄, identiques ou différents, représentent un atome d'hydrogène, un groupe hydroxyle, un groupe (C₁-C₆) alkyle linéaire ou ramifié, un groupe (C₁-C₆) alkoxy linéaire ou ramifié, un groupe -OCO-alkyle en (C₁-C₆) linéaire ou ramifié,
- R₅ représente un atome d'hydrogène ou un groupe (C₁-C₆) alkyle linéaire ou ramifié,
- m est un nombre entier compris entre 0 et 2, et
- n est un nombre entier compris entre 8 et 25.

2. Composés selon la revendication 1, **caractérisés en ce que** n est un nombre entier compris entre 8 et 16.

3. Composés selon la revendication 2, **caractérisés en ce que** n est un nombre entier égal à 8, 10, 12, 13, 14 ou 16.

4. Composés selon la revendication 1, **caractérisés en ce que** le composé est un composé comprenant une chaîne latérale d'au moins 12 atomes de carbone et d'au plus 18 atomes de carbone.

5. Composés selon l'une quelconque des revendications précédentes, **caractérisé en ce que** R₅ représente un atome d'hydrogène ou un radical méthyle.

6. Composés selon l'une quelconque des revendications précédentes, **caractérisés en ce que** l'atome de carbone portant le substituant R₅ est de configuration R, S ou un mélange

7. Composés de formule (I) selon l'une quelconque des revendications précédentes, **caractérisés en ce que** au moins un, de préférence un seul, des substituants présents sur le noyau aromatique, R₁, R₂, R₃ et/ou R₄ représente un groupe hydroxyle, alkoxy ou carboxylate.

8. Composés de formule (I) selon l'une quelconque des revendications précédentes, **caractérisés en ce que** le groupe (C₁-C₆) alkyle linéaire ou ramifié est le radical méthyle, éthyle, isopropyle ou tertiobutyle.

9. Composés de formule (I) selon l'une quelconque des revendications 1 à 7, **caractérisés en ce que** le groupe (C₁-C₆) alkoxy linéaire ou ramifié est le groupe méthoxy, éthoxy, isopropoxy ou tert-butoxy.

10. Composition pharmaceutique, **caractérisée en ce qu'**elle comprend au moins un composé selon l'une des revendications 1 à 9, en association avec un véhicule acceptable sur le plan pharmaceutique.

11. Composition pharmaceutique comprenant à titre de substance active au moins un composé selon la revendication 1 provoquant la modulation de la spécification des cellules souches neurales et/ou la différentiation de cellules précurseurs d'oligodendrocytes en cellules oligodendrogliales et/ou réprimant l'activation des cellules microgliales et/ou l'activation des astrocytes et/ou la gliose réactionnelle, en association avec un véhicule acceptable sur le plan pharmaceutique.

12. Utilisation d'au moins un composé selon la revendication 1
dans le cadre de la préparation d'une composition pharmaceutique destinée à la prévention ou au traitement des maladies du système nerveux altérant les oligodendrocytes ou les autres cellules du système nerveux et/ou de l'inflammation du système verveux

13. Utilisation d'au moins un composé selon la revendication 1,
dans le cadre de la préparation d'une composition pharmaceutique destinée à la prévention ou au traitement des neuropathies dégénératives.

14. Utilisation selon la revendication 13, **caractérisée en ce que** la composition pharmaceutique est destinée à la prévention ou au traitement des maladies demyélinisantes ou dysmyélinisantes.

15. Utilisation selon la revendication 13 ou 14, **caractérisée en ce que** la composition pharmaceutique prévient ou traite la sclérose en plaques, la maladie d'Alzheimer, la maladie de Parkinson, la maladie de Creutzfeldt-Jakob, les accidents vasculaires cérébraux ou toutes autres atteintes lésionnelles du système nerveux.

16. Utilisation d'au moins un composé selon l'une quelconque des revendications précédentes 1-9 pour la préparation d'une composition pharmaceutique destinée à moduler, *in vivo* ou *in vitro,* la spécification cellulaire des cellules souches neurales, à favoriser la différenciation puis la survie des neurones et cellules gliales en différenciation, à favoriser la différenciation de cellules précurseurs d'oligodendrocytes en oligodendrocytes matures, et/ou à diminuer l'activation de la microglie et/ou l'activation des ashrocytes et/ou la gliose réactionnelle.

17. Procédé de préparation d'un composé de formule (I) selon l'une des revendications 1 à 9, **caractérisé en ce qu'**il met en oeuvre les étapes du schéma réactionnel suivant : 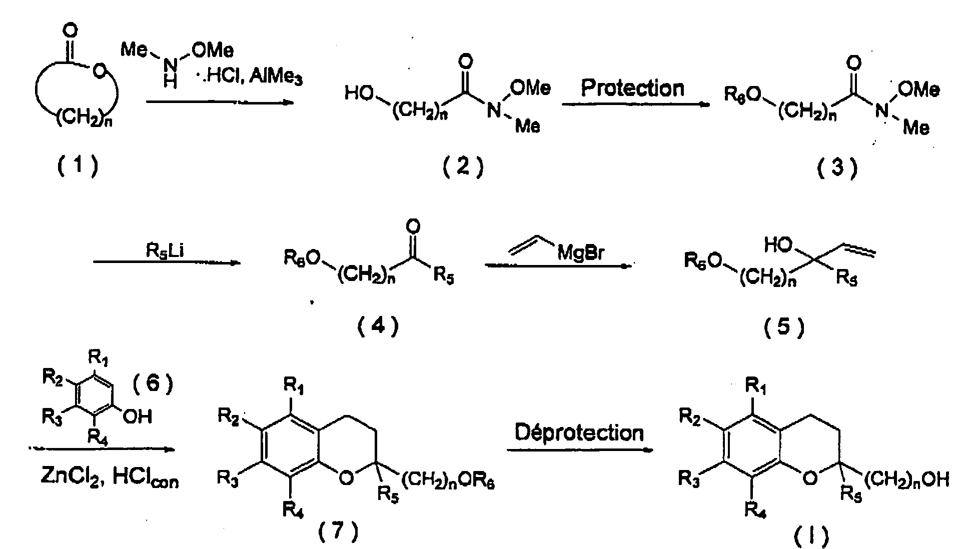 dans lequel :
- R₁, R₂, R₃, R₄, R₅ et n ont la même signification que décrite dans la revendication 2 et
- R₆ représente un groupement benzyle ou un groupement *tert*-butyldiméthylsilyle.

## Claims

1. Compounds of the general Formula (I): 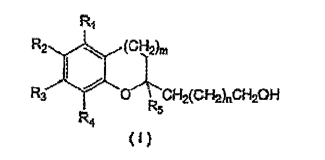 wherein
- R₁, R₂, R₃ and R₄, identical or different, represent a hydrogen atom, a hydroxyl group, a linear or branched (C₁-C₆) alkyl group, a linear or branched (C₁-C₆) alkoxy group, a linear or branched -OCO-(C₁-C₆) alkyl group,
- R₅ represents a hydrogen atom or a linear or branched (C₁-C₆) alkyl group,
- m is an integer between 0 and 2, and
- n is an integer between 8 and 25

2. . Compounds according to Claim 1, wherein n is an integer between 8 and 16

3. . Compounds according to Claim 2, wherein n is an integer equal to 8, 10, 12, 13, 14 or 16.

4. . Compounds according to Claim 1, wherein the compound is a compound comprising a lateral chain of at least 12 carbons and at most 18 carbons.

5. . Compounds according to any of the preceding Claims, wherein R₅ represents a hydrogen atom or a methyl group.

6. Compounds according to any of the preceding Claims, wherein the carbon atom bearing the substituent R₅ has the R or S configuration or is a mixture

7. Compounds of Formula (I) according to any of the preceding Claims, wherein at least one, preferably only one, of the substituents R₁, R₂, R₃ and R₄ on the aromatic ring, represents a hydroxyl, alkoxy or carboxylate group.

8. . Compounds of Formula (I) according to any of the preceding Claims, wherein the linear or branched C₁-C₆ alkyl group is the methyl, ethyl, isopropyl or tert -butyl radical.

9. Compounds of Formula (I) according to any of Claims 1 to 7, wherein the linear or branched (C₁-C₆) alkoxy group is the methoxy, ethoxy, isopropoxy or *tert*.-butoxy group

10. . Pharmaceutical composition comprising at least one compound according to one of Claims 1 to 9, in association with a pharmaceutically acceptable carrier

11. Pharmaceutical composition comprising at least one compound according to claim 1 as the active principle, said composition
stimulating the modulation of the specification of the neural stem cells and/or the differentiation of cells that are precursors of oligodendrocytes in oligodendroglial cells and/or repressing the activation of microglial cells and/or the activation of astrocytes and/or reactive gliosis, in association with a pharmaceutically acceptable carrier

12. Use of at least one compound according to claim 1 in the preparation of a pharmaceutical composition for the prevention or treatment of diseases of the nervous system altering the oligodendrocytes or the other cells of the nervous system and/or the inflammation of the nervous system

13. Use of at least one compound according to claim 1, in the preparation of a pharmaceutical composition for the prevention or the treatment of degenerative neuropathies

14. Use according to Claim 13, wherein the pharmaceutical composition is intended for the prevention or the treatment of demyelinising or dysmyelinising diseases.

15. Use according to Claim 13 or 14, wherein the pharmaceutical composition prevents or treats multiple sclerosis, Alzheimer's disease, Parkinson's disease, Creutzfeldt-Jakob disease, cerebral vascular accidents or any other damaging attacks to the nervous system

16. Use of at least one compound according to one of the preceding Claims 1-9 for the preparation of a pharmaceutical composition intended to modulate, *in vivo* or *in vitro,* the cellular specification of the neural stem cells, to favour the differentiation and then the survival of the neurones and glial cells in differentiation, to favour the differentiation of precursor cells of oligodendrocytes in mature oligodendrocytes, and/or to reduce the activation of the microglia and/or the activation of the astrocytes and/or the reactive gliosis.

17. Process for preparing a compound of Formula (I) according to one of the Claims 1 to 9, wherein said process includes the reaction steps of the following scheme: 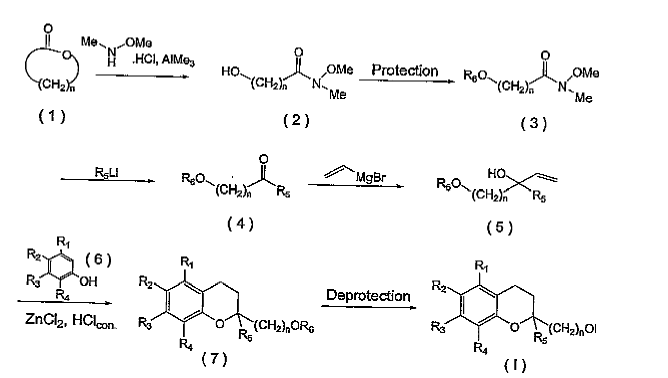 wherein
- R₁, R₂, R₃, R₄, R₅ and n have the same meaning as described in Claim 2 and
- R₆ represents a benzyl group or a *tert*-butyldimethylsilyl group.

## Patentansprüche

1. Verbindungen, **dadurch gekennzeichnet, dass** sie die folgende allgemeine Formel (I) aufweisen: 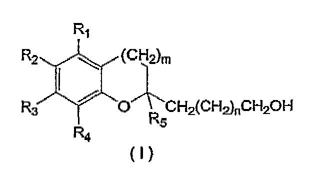 in der
- R₁, R₂, R₃ und R₄, die gleich oder verschieden sind, für ein Wasserstoffatom, eine Hydroxylgruppe, eine geradkettige oder verzweigte (C₁-C₆)-Alkylgruppe, eine geradkettige oder verzweigte (C₁-C₆)-Alkoxygruppe, eine geradkettige oder verzweigte OCC-(C₁-C₆)-Alkylgruppe stehen,
- R₅ für ein Wasserstoffatom oder eine geradkettige oder verzweigte (C₁-C₆)-Alkylgruppe steht,
- m eine ganze Zahl zwischen 0 und 2 ist und
- n eine ganze Zahl zwischen 8 und 25 ist

2. Verbindungen nach Anspruch 1, **dadurch gekennzeichnet, dass** n eine ganze Zahl zwischen 8 und 16 ist.

3. Verbindungen nach Anspruch 2, **dadurch gekennzeichnet, dass** n eine ganze Zahl ist, die gleich 8, 10, 12, 13, 14 oder 16 ist

4. Verbindungen nach Anspruch 1, **dadurch gekennzeichnet, dass** es sich bei der Verbindung um eine Verbindung handelt, die eine Seitenkette von mindestens 12 Kohlenstoffatomen und höchstens 18 Kohlenstoffatomen umfasst.

5. Verbindungen nach einem der vorhergehenden Ansprüche, **dadurch gekennzeichnet, dass** R₅ für ein Wasserstoffatom oder einen Methylrest steht

6. Verbindungen nach einem der vorhergehenden Ansprüche, **dadurch gekennzeichnet, dass** das Kohlenstoffatom den Substituenten R₅ in R- oder S-Konfiguration oder einer Mischung davon trägt

7. Verbindungen der Formel (I) nach einem der vorhergehenden Ansprüche, **dadurch gekennzeichnet, dass** mindestens einer, vorzugsweise ein einziger der Substituenten, die in dem aromatischen Kern vorliegen, R₁, R₂, R₃ und/oder R₄, für eine Hydroxyl-, Alkoxy- oder Carboxylatgruppe steht

8. Verbindungen der Formel (I) nach einem der vorhergehenden Ansprüche, **dadurch gekennzeichnet, dass** es sich bei der geradkettigen oder verzweigten (C₁-C₆)-Alkylgruppe um den Methyl-, Ethyl-, Isopropyl- oder tert -Butylrest handelt

9. Verbindungen der Formel (I) nach einem der Ansprüche 1 bis 7, **dadurch gekennzeichnet, dass** es sich bei der geradkettigen oder verzweigten (C₁-C₆)-Alkoxygruppe um die Methoxy-, Ethoxy-, Isopropoxy- oder tert-Butoxygruppe handelt

10. Pharmazeutische Zusammensetzung, **dadurch gekennzeichnet, dass** sie mindestens eine Verbindung nach einem der Ansprüche 1 bis 9 zusammen mit einem pharmazeutisch unbedenklichen Trägerstoff umfasst.

11. Pharmazeutische Zusammensetzung, die als Wirkstoff mindestens eine Verbindung nach Anspruch 1 umfasst, die die Modulation der Spezifizierung von Nervenstammzellen und/oder die Differenzierung von Oligodendrozytenvorläuferzellen in Oligodendrogliazellen bewirkt und/oder die Aktivierung von Mikrogliazellen und/oder die Aktivierung von Astrozyten und/oder die reaktive Gliose unterdrückt, zusammen mit einem pharmazeutisch unbedenklichen Trägerstoff

12. Verwendung mindestens einer Verbindung nach Anspruch 1 im Rahmen der Herstellung einer pharmazeutischen Zusammensetzung, die zur Verhinderung oder Behandlung von Erkrankungen des Nervensystems, die die Oligodendrozyten oder die anderen Zellen des Nervensystems modifizieren, und/oder einer Entzündung des Nervensystems gedacht ist

13. Verwendung mindestens einer Verbindung nach Anspruch 1 im Rahmen der Herstellung einer pharmazeutischen Zusammensetzung, die zur Verhinderung oder Behandlung von degenerativen Neuropathien gedacht ist.

14. Verwendung nach Anspruch 13, **dadurch gekennzeichnet, dass** die pharmazeutische Zusammensetzung zur Verhinderung oder Behandlung von demyelinisierenden oder dysmyelinisierenden Erkrankungen gedacht ist

15. Verwendung nach Anspruch 13 oder 14, **dadurch gekennzeichnet, dass** die pharmazeutische Zusammensetzung multiple Sklerose, Alzheimer-Krankheit, Parkinson-Krankheit, Creuzfeldt-Jakob-Krankheit, Schlaganfälle oder alle anderen Läsionen des Nervensystems verhindert oder behandelt

16. Verwendung mindestens einer Verbindung nach einem der vorhergehenden Ansprüche 1 - 9 bei der Herstellung einer pharmazeutischen Zusammensetzung, die zum Modulieren der zellulären Spezifizierung von Nervenstammzellen *in vivo* oder *in vitro,* Fördern der Differenzierung und anschliessend des Überlebens von Neuronen und Gliazellen die sich im Differenzierungsprozess befinden, Fördern der Differenzierung von Oligodendrozytenvorläuferzellen in reife Oligodendrozyten und/oder Einschränken der Aktivierung der Mikroglia und/oder der Aktivierung von Astrozyten und/oder der reaktiven Gliose gedacht sind

17. Verfahren zur Herstellung einer Verbindung der Formel (I) nach einem der Ansprüche 1 bis 9, **dadurch gekennzeichnet, dass** es die Schritte des folgenden Reaktionsschemas ausführt: 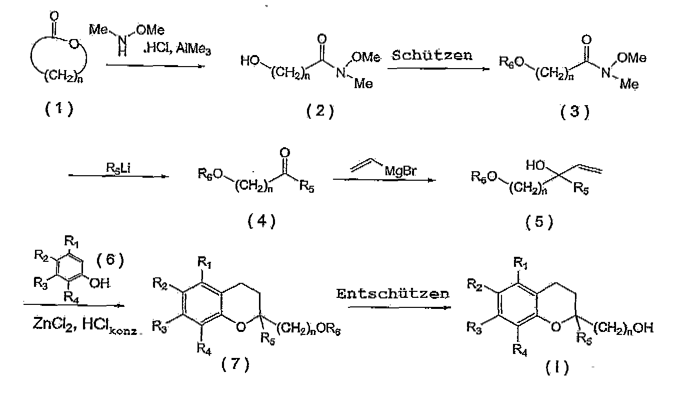 in der
- R₁, R₂, R₃, R₄, R₅ und n dieselbe Bedeutung haben, wie in Anspruch 2 beschrieben wurde, und
- R₆ für eine Benzylgruppe oder eine *tert*.-Butyldimethylsilylgruppe steht.
